# EUROPEAN PATENT SPECIFICATION

(11) **EP 2 890 344 B1**
(45) Date of publication and mention of the grant of the patent: **23.09.2020**
(21) Application number: 12883607.9
(22) Date of filing: 28.08.2012
(51) Int. Cl.: A61F 13/42, H04M 11/04, A61B 5/20, G08B 21/20

(54) **METHOD AND MOBILE APPLICATIONS USING CROSS-SHARING DATABASE FOR MONITORING USE OF HYGIENE PRODUCTS**
VERFAHREN UND MOBILE ANWENDUNGEN UNTER VERWENDUNG EINER ÜBERGREIFENDEN DATENBANK ZUR ÜBERWACHUNG VON HYGIENEPRODUKTEN
PROCÉDÉ ET APPLICATIONS MOBILES UTILISANT UNE BASE DE DONNÉES DE PARTAGE CROISÉ POUR SURVEILLER L'UTILISATION DE PRODUITS D'HYGIÈNE

(43) Date of publication of application: 08.07.2015
(73) Proprietor: Essity Hygiene and Health Aktiebolag, 405 03 Göteborg (SE)
(72) Inventor: CARNEY, Joshua, 405 03 Göteborg (SE); CARLÉN, Henrik, 405 03 Göteborg (SE)
(74) Representative: Hoffmann Eitle
(86) International application number: PCT/SE2012/050910
(87) International publication number: WO 2014/035302

(56) References cited:
- WO-A1-01/50996
- WO-A1-2008/023289
- WO-A1-2008/147612
- WO-A1-2009/027871
- WO-A1-2011/008838
- WO-A1-2011/125003
- JP-A- 2004 212 060
- US-A1- 2007 270 774
- US-A1- 2012 040 655

## Description

### TECHNICAL FIELD

The present invention relates to a method, computer program and mobile device for monitoring use of a hygiene product, where information related to the use of the hygiene product is provided to the product-wearer or a caregiver of the product-wearer based on information related to the product-wearer's voiding behaviour.

### BACKGROUND

Today, it is common for both children and adults to use various types of hygiene products such as diapers, incontinence pads, sanitary napkins etc.

Users of incontinence products may sometimes have difficulty in selecting the correct type of product on the market of incontinence products, i.e. selecting the product which is best suited for the user's individual needs. This is emphasized by the fact that users of incontinence products are often elderly or disabled persons.

Improper monitoring and change of absorbent products may cause urinary and faecal leakage from the product. To many people suffering from incontinence, this is a huge problem often causing feelings of shame and humiliation.

For this reason, there is a need for methods and devices to aid such users in monitoring their use of hygiene products, and in finding suitable hygiene products that are adapted for their own needs.

Several solutions for improved monitoring of use of absorbent articles are known from prior art.

US 2007/0252713 discloses an absorbent sensor pad worn by a patient. One or more sensors that measure urinary voiding parameters are integrally formed in the pad. The sensors may include impedance sensors, strain gauges, temperature sensors, accelerometers, pH sensors, and chemical sensors that measure wetness, volume, temperature, pH, and contents of urine voided by a patient as well as the posture and activity of the patient. The voiding data sensed by the sensors may be stored in a voiding log which may be transmitted to an external device connected to the sensors.

US 2009/0062758 relates to a wetness monitoring system for e.g. a diaper. The system includes a wetness sensor capable of counting the number of discrete insults, and an alarm that is triggered after a critical number of insults, or when a certain period of time has elapsed since the last change of product.

US 2011/0263952 discloses an incontinence management system for monitoring wetness in absorbent articles. The system comprises input for receiving sensor signals indicative of a presence of wetness in an absorbent article and a user interface for communicating with a user of the system.

US 2007/270774 A1 discloses an incontinence management system for monitoring wetness in one or more absorbent articles.

US 2012/040655 A1 discloses a cellular phone based systems for monitoring sensor signals of sensors in vaginally inserted tampons.

WO 2011/125003 A1 discloses a sensing apparatus and a method for predicting urination.

However, known solutions for monitoring use of absorbent articles often involve complex and expensive products and/or monitoring systems that are not readily available to the public.

### SUMMARY OF THE INVENTION

It is an objective of the present invention to solve or at least mitigate one or more of the above mentioned problems.

In particular, it is an objective of the present invention to provide a cost efficient and readily available method for monitoring use of hygiene products.

Another objective of the invention is to provide a method that can help preventing too late change of absorbent products.

These and other objectives are also addressed in the co-pending patent applications PCT/SE2011/051 565 (WO2013/095230), PCT/SE2011/051 566 (WO2013/095231), PCT/SE2011/051551, (WO2013/095222) , and PCT/SE2011/051 558 (WO2013/095226), filed by the Applicant of the present patent application in December 2011 and not available to the public at the date of filing of this application. These co-pending applications describe inventions relating to different mobile applications (so called "apps") for monitoring use of absorbent products, such as incontinence pads. The mobile applications all collect information that is relevant to the voiding behaviour of a wearer of such a product in order to provide product-related information to the product-wearer as to the use of the absorbent product, based on the collected information. The collected information may be information indicative of actual urinary and/or faecal insults, or it may be other information that affects or potentially affects the voiding behaviour of the product-wearer.

In some basic versions of some of these mobile applications, the collected information may contain an indication that voiding has taken place, whereby the product-related information may comprise a notification that there might be a need for changing the absorbent product, displayed on the product-wearer's mobile device.

In more refined versions of some of these mobile applications, they are adapted to obtain "capacity information" related to the capacity of the absorbent product worn by the wearer, and to present recommendations as to the use of the absorbent product based on a comparison between the voiding behaviour of the wearer, as determined from the collected information, and the capacity of the absorbent product. If, for example, the collected information indicates that the product-wearer has had a certain number of insults of urine and/or faeces that are well below the capacity of the absorbent product, the product-related information provided to the product-wearer may include information indicating that no change of the absorbent product is required at the moment. If, however, the collected information indicates that the product-wearer has had a number of insults of urine and/or faeces that is close to the maximum capacity of the absorbent product, the product-related information may include a recommendation to change the product. If it is found that the capacity of the absorbent product is too high or too low for the voiding behaviour of the product-wearer, the product-related information may comprise a recommendation to exchange the product for another product having a higher or lower absorbing capacity, displayed on the product-wearer's mobile device.

The present invention relates to a method that improves these and any other mobile applications for monitoring use of hygiene products by providing a solution allowing such a mobile application to provide the product-related information based also on information that is obtained by other mobile applications but still relevant to the voiding behaviour of the product-wearer. By incorporating into a mobile application for monitoring use of hygiene products the functionality of retrieving information from a database storing information that is relevant to the voiding behaviour of the product-wearer, obtained through the use of other mobile applications, more information affecting the use of the hygiene product can be obtained by the mobile application, and so more relevant product-related information can be provided to the product-wearer. The information retrieved from the database may be used to increase the intelligence of the mobile application monitoring the use of the hygiene product, e.g. by adapting an algorithm used by the mobile application to predict future voiding by the product-wearer based on the retrieved information.

The invention hence provides a method as defined in claim 1.

Thus, the invention suggests the use of a "cross-sharing database" from which information that is relevant to the voiding behaviour of a user, obtained by means of a mobile application, can be retrieved by another mobile application for monitoring the use of a hygiene product worn by said user.

The information relevant to the voiding behaviour of the product-wearer may include data on the actual voiding behaviour of the product-wearer, e.g. the time, type and amount of one or more urinary and/or faecal insults, and/or data on the product-wearer himself or other behaviours or routines of the product-wearer that affect or potentially affect the product-wearer's voiding behaviour. Non-exclusive examples of voiding relevant information that affects or potentially affects the voiding behaviour of the product-wearer and that may be stored in and retrieved from the cross-sharing database are:
- data on the age and gender of the product-wearer (user data);
- data on the health and use of medicines of the product-wearer (health data);
- data on intakes of fluid and/or solid edible material by the product-wearer (intake data);
- data on exercise routines of the product-wearer (exercise data), and
- data on sleep routines of the product-wearer (sleep data).

The first mobile application may further be configured to collect voiding-related information on the actual voiding behaviour of the product-wearer, and to provide the product-related information based on a comparison between the voiding-related information and the information retrieved from the cross-sharing database. The voiding-related information may be collected by the first mobile application through manual input of information by the product-wearer, and/or by means of one or more sensor devices capable of obtaining information indicative of actual voiding of urine and/or faeces by the product-wearer.

The first mobile application is configured to predict a future voiding behaviour of the product-wearer based on the information retrieved from the cross-sharing database, and to provide the product-related information based on the predicted future voiding behaviour. The prediction may be based on a comparison between voiding-related information obtained by the first mobile application itself and the information retrieved from the cross-sharing database.

As mentioned above, the information retrieved from the cross-sharing database may be information on behaviours or routines of the product-wearer affecting the voiding behaviour of the product-wearer, e.g. information on the intake behaviour, the exercise behaviour and/or the sleep behaviour of the product-wearer. By comparing this information with voiding-related information obtained by the first mobile application itself, the first mobile application can learn how other behaviours and routines of the product-wearer affect the product-wearer's voiding behaviour. This knowledge may then be used by the first mobile application in the prediction of future voiding by the product-wearer, and so makes the first mobile application capable of providing product-related information in form of useful recommendations as to the use of the hygiene product to the product-wearer or a caregiver of the product-wearer. Using a slightly different wording, the first mobile application can be said to determine a correlation between the voiding behaviour of the product-wearer and other behaviours and routines of the product-wearer, which correlation is used to predict a future voiding behaviour of the product-wearer.

The first and the at least second mobile applications thus form a set of mobile applications which together serve to analyse how different behaviours and routines of the product-wearer affect his/her voiding behaviour, so as to provide as useful recommendations as possible as to the use of hygiene products.

Preferably, the first and the at least second mobile applications are members of a group of mobile applications all configured to monitor the use of a hygiene product worn by a wearer. These mobile applications may all be configured to obtain voiding relevant information on the product-wearer, and to transmit the obtained information to the cross-sharing database. Some or all of the mobile applications may further be configured to retrieve voiding relevant information obtained by the other mobile applications from the cross-sharing database, and to use this information to provide relevant product-related information concerning the use of hygiene products to the product-wearer.

The first and the at least second mobile applications may for example be different applications from a group comprising:
1) a mobile application, hereinafter referred to as "Sleep Security", wherein information relevant to the voiding behaviour of a user is obtained by analysing the movements of the user, registered by means of a movement sensing device of a mobile device, similar to the mobile application described in PCT/SE2011/051565
2) a mobile application, hereinafter referred to as "Daily Log", wherein information relevant to the voiding behaviour of a user is obtained through manual input of at least information related to intakes of fluid and/or solid edible material by the user, and information related to voiding of urine and/or faeces by the user, similar to the mobile application described in PCT/SE2011/051566
3) a mobile application, hereinafter referred to as "Pad Scanner", wherein information relevant to the voiding behaviour of a user is obtained by analysing images of used absorbent products, captured by an image capturing device of a mobile device, similar to the mobile application described in PCT/SE2011/051551, and
4) a mobile application, hereinafter referred to as "Reassure", wherein information relevant to the voiding behaviour of a user is obtained by analysing signals received from an odour sensor device capable of detecting chemicals indicative of a presence of urine and/or faeces, similar to the mobile application described in PCT/SE2011/051558.

In one embodiment, the cross-sharing database is particularly intended for exchange of voiding relevant information between the Sleep Security and the Daily Log applications. For example, the Sleep Security application may be configured to provide voiding relevant information related to the registered movement of the user to the cross-sharing database, whereby the Daily Log application may be configured to use this information together with the information manually input by the user to the Daily Log application in order to more accurately predict future insults of urine and/or faeces by the product-wearer.

The cross-sharing database may be a single-user database from which a user of the first mobile application only can retrieve voiding relevant information concerning his or hers own person and behaviours. In other embodiments, the cross-sharing database is a multi-user database, meaning that the first mobile application can retrieve voiding relevant information concerning other users of the at least one second mobile application. Thereby, information on how personal data, behaviours and routines of other users affect their voiding behaviours can be retrieved from the database by the first mobile application and used to predict the future voiding behaviour of the product-wearer.

Preferably, the first mobile application is further configured to obtain capacity information related to the capacity of the hygiene product worn by the user of the mobile application, and to provide the product-related information based on both voiding-related information (obtained by the first mobile application itself or retrieved from the cross-sharing database) and said capacity information. This allows the first mobile application to provide product-related information in form of well-founded recommendations as to the use of the hygiene product, as the product-related information can be based on a comparison between the voiding behaviour of the product-wearer and the capacity of the hygiene product.

The capacity information may comprise any of, or any combination of the type of the absorbent product, the absorbency level of the absorbent product, and the size of the hygiene product. The capacity information may be obtained from user input and/or reception of information from a product database which may or may not form part of the cross-sharing database.

As mentioned above, the first mobile application may be configured to use the voiding relevant information retrieved from the cross-sharing database to predict future insults of urine and/or faeces by the product-wearer. It may also be configured to predict the amount of urine and/or faeces likely to be voided in the future based on the retrieved information and, additionally, voiding-related information obtained by the first mobile application itself, and to provide the product-related information based on the amount of urine and/or faeces predicted to be voided in the future.

As also mentioned above, the first mobile application may be configured to obtain capacity information related to the capacity of the hygiene product, in which case the product-related information can be provided based on a comparison between the capacity of the hygiene product and the amount of urine and/or faeces predicted to be voided in the future.

For example, the first mobile application may be configured to determine a maximum amount of urine and/or faeces that can be retained by the hygiene product based on said capacity information, and to determine, for a future point in time, the amount of urine and/or faeces likely to be voided by the product-wearer at that point in time, based on said predicted future voiding behaviour of the product-wearer. The recommendation can then be based on a comparison between said maximum amount and the amount of urine and/or faeces likely to be voided at said future point in time. The maximum amount of urine and/or faeces that can be retained by the hygiene product and the amount of urine and/or faeces likely to be voided at the future point in time are preferably determined by the first mobile application as discrete numbers of insults of urine and/or faeces. If the amount voided at the future point in time exceeds or is close to the maximum amount that can be retained by the hygiene product, the product-related information provided by the first mobile application may be a recommendation to change the hygiene product before that future point in time.

In the above scenario, the product-related information provided by the first mobile application comprises a recommendation to change the product before the voided amount exceeds the amount that can be retained by the product. In general, however, the product-related information may be any information concerning the use of the hygiene product. For example, the product-related information may comprise any of, or any combination of: an indication that no change of product is necessary; an indication that urinary and/or faecal voiding has taken place and that it may be advisable to change the product; a recommendation to change the product; a recommendation to change the product before a certain time; and, a recommendation to exchange the product for another hygiene product having higher or lower capacity than the currently worn product.

In an exemplary embodiment of the invention, the first mobile application is the Daily Log application which obtains information on the voiding and intake behaviours of the product-wearer through manual input of information into a daily log, and the at least second mobile application is the Sleep Security application which obtains voiding-related information on the voiding behaviour of the product-wearer during night time by analysing the product-wearer's sleeping movements. The Daily Log application retrieves the voiding-related information obtained by the Sleep Security application from the cross-sharing database and analyses the relationship between intake and voiding by the user during daytime and the voiding behaviour of the user during night time. Based on this analysis, the Daily Log application can learn to predict the voiding behaviour of the product-wearer during a night following a day having a certain intake/voiding pattern. The Daily Log application may then provide the product-related information to the product-wearer based on this prediction. For example, Daily Log may display a notification to the product-wearer in the evening that it is likely that the hygiene product will have to be changed before a certain point in time during the night to come, in order to avoid urinary leakage from the product.

The first mobile application can be configured to provide the product-related information to the product-wearer by means of visual, audible and/or vibratory signalling on the mobile device, and/or on a communication device to which the mobile device is communicatively connectable, e.g. a mobile device of a caregiver of the product-wearer. Preferably, both the content of the product-related information and the way the product-related information is provided to the product-wearer and/or the caregiver is determined based at least partly on the voiding relevant information retrieved from the cross-sharing database.

As is clear from the above description, the method according to the invention is a computer-implemented method performed by set of computer programs in form of mobile applications for monitoring use of a hygiene product worn by a wearer, which set of computer programs is formed of a first mobile application and at least a second mobile application, wherein the set of computer programs are configured to share the information stored in a database, and the computer programs, when executed by a processor of one or more mobile devices, causes the one or more mobile devices to perform the method as defined above.

The invention also provides a computer-readable data carrier having stored thereon the set of computer programs defined above, and a database configured to be shared by the first mobile application and the at least a second mobile application..

Furthermore, the invention provides a mobile device for monitoring use of a hygiene product worn by a wearer. The mobile device comprises a processor and a storage medium for storing computer programs executable by said processor, which storage medium stores the above mentioned computer program. The mobile device further comprises a communication unit for connecting to and retrieving the voiding relevant information from the cross-sharing database.

Preferably, the mobile device further comprises:
- a movement sensing device, such as an accelerometer, for registering the movements of the product-wearer when the mobile device is carried by the product-wearer or placed next to the product-wearer in bed;
- input means allowing a user to manually input information relating to the intake behaviour and the voiding behaviour of the user;
- an image sensing device, such as an integrated camera, for capturing images of used absorbent products, e.g. incontinence pads, and
- a communication unit for communicating with an odour sensor device configured to detect at least one chemical indicative of a presence of urine and/or faeces in a hygiene product.

### BRIEF DESCRIPTION OF THE DRAWINGS

The present invention will become more fully understood from the detailed description provided hereinafter and the accompanying drawings which are given by way of illustration only. In the different drawings, same reference numerals correspond to the same element.

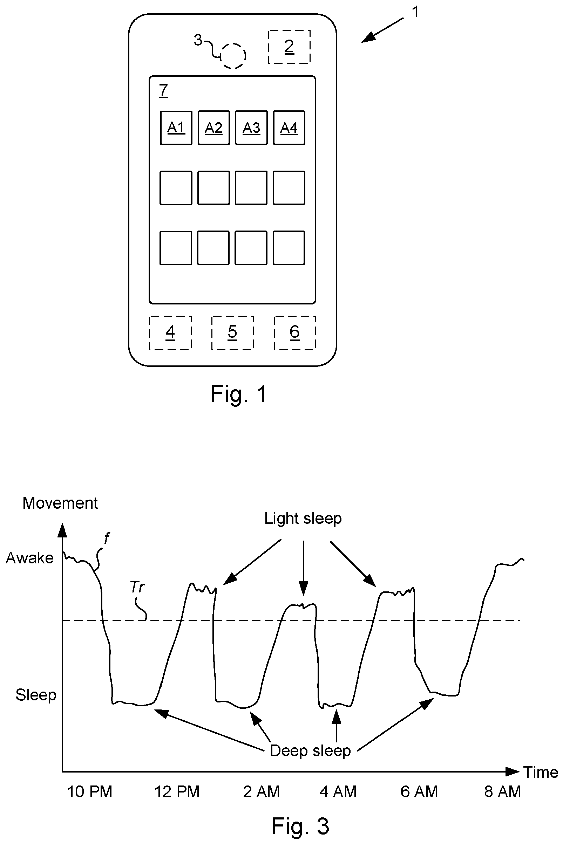

Fig. 1 illustrates a mobile device storing a plurality of mobile applications for monitoring use of hygiene products.

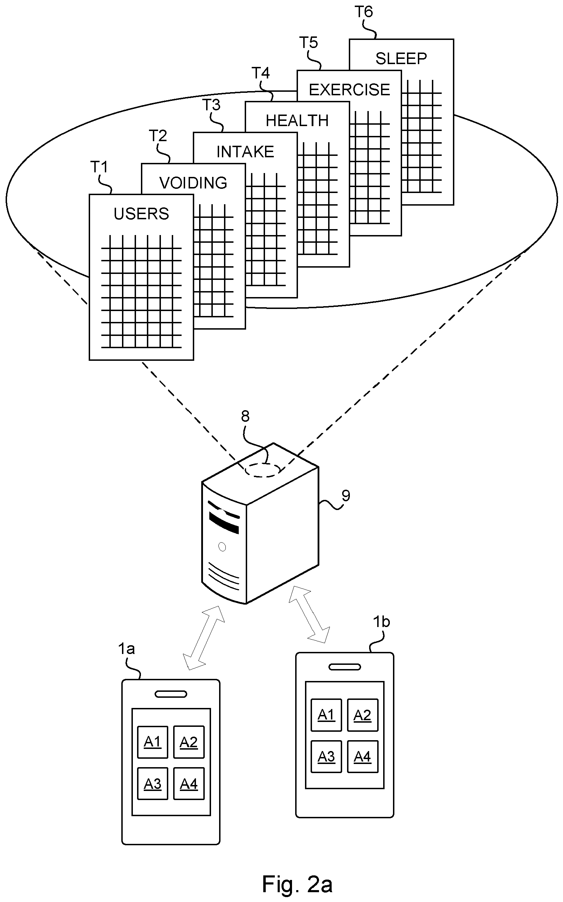

Fig. 2a illustrates a system for monitoring use of a hygiene product according to an exemplary embodiment of the invention.

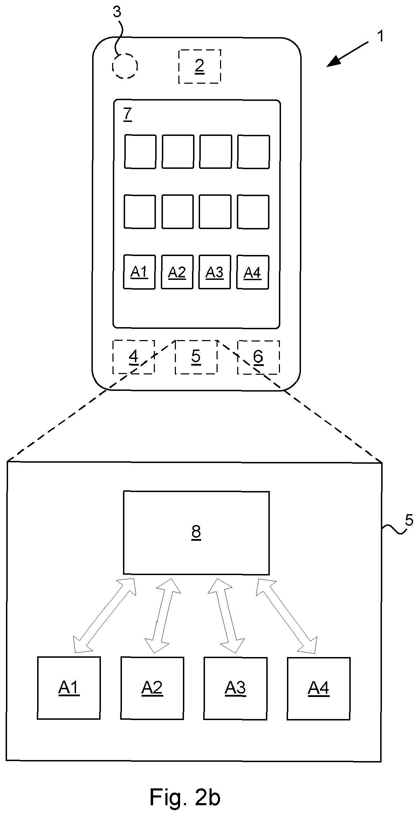

Fig. 2b illustrates a system for monitoring use of a hygiene product according to another exemplary embodiment of the invention.
Fig. 3 illustrates an "awakeness curve" that can be obtained by means of a movement sensing device of a mobile device and analysed by a mobile application to provide product-related information as to the use of a hygiene product to a wearer of the hygiene product, and/or to transmit information that is relevant to the voiding behaviour of the user to a cross-sharing database for subsequent retrieval and use by other mobile applications.

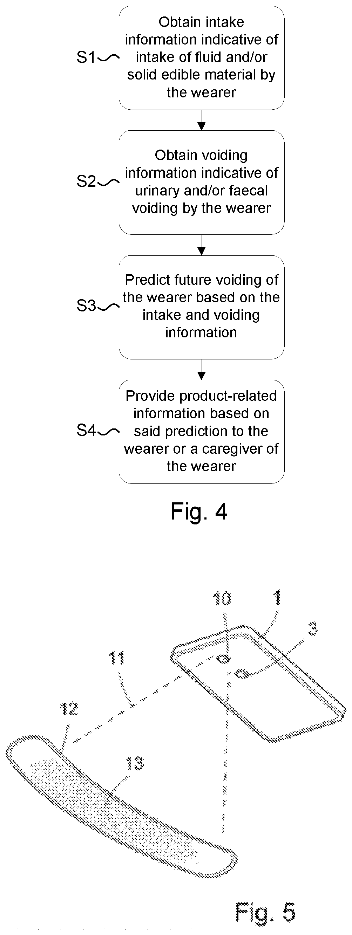

Fig. 4 illustrates method steps performed by a mobile application to provide product-related information as to the use of a hygiene product to a wearer of the hygiene product, and/or to transmit information that is relevant to the voiding behaviour of the product-wearer to a cross-sharing database for subsequent retrieval and use by other mobile applications.
Fig. 5 illustrates a process of capturing an image of a used hygiene product, which image can be analysed by a mobile application to provide product-related information as to the use of a hygiene product to a wearer of the hygiene product, and/or to transmit information that is relevant to the voiding behaviour of the product-wearer to a cross-sharing database for subsequent retrieval and use by other mobile applications.

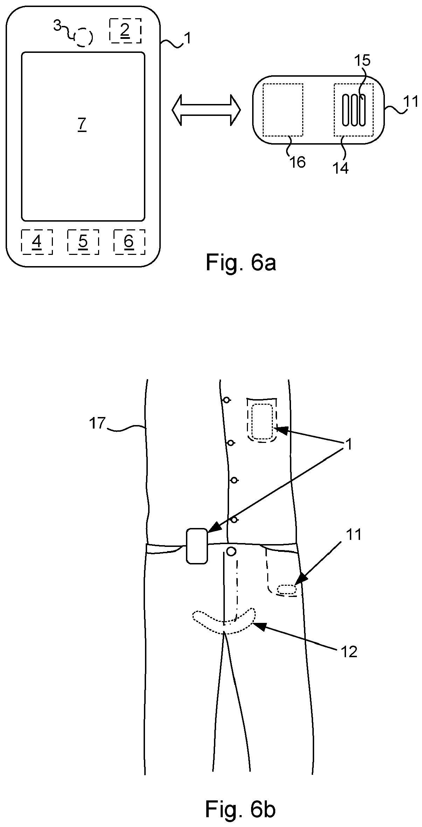

Figs. 6a and 6b illustrates a system employing an odour sensor device which can be used to send voiding-related information related to the voiding behaviour of a wearer of a hygiene product to a mobile application. The mobile application can analyse the received information to provide product-related information as to the use of a hygiene product to a wearer of the hygiene product, and/or to transmit information that is relevant to the voiding behaviour of the product-wearer to a cross-sharing database for subsequent retrieval and use by other mobile applications.

### DETAILED DESCRIPTION

Fig. 1 illustrates a mobile device 1 for performing the method according to the invention. The mobile device 1 in Fig. 1 is a mobile phone in form of what is often referred to as a smartphone but it should be appreciated that the mobile device according to the invention may be any type of hand-held computing device, such as a personal digital assistant (PDA) or a tablet computer, devised and configured as set forth below.

The mobile device 1 comprises a movement sensing device 2 in form of an integrated accelerometer for registering motion data indicative of the movement of the mobile device. Such accelerometers are well known in the art and used in many mobile devices of today. The accelerometer 2 is operable to register the movement of a person carrying the mobile device, and preferably also to register the movements of a sleeping person when the mobile device 1 is placed in bed next to the sleeping person. Although the movement sensing device 2 in a preferred embodiment of the invention is realised in form of an integrated accelerometer of the mobile device 1, other types of movement sensing devices may be used instead or in addition to an accelerometer. For example, the movement sensing device may include a gyroscope of the mobile device 1.

Furthermore, the mobile device 1 comprises an integrated image capturing device 3 for capturing images and video recordings to be subsequently stored in the mobile device 1. Such image capturing devices 3 are generally known and typically comprise an image sensor and a lens (not shown) which are used for example in mobile phones for capturing photographic images.

The mobile device 1 further comprises a processor 4 for processing data. The data may be received from communication devices to which the mobile device 1 is communicatively connectable via a network, or stored on a digital storage medium 5 of the mobile device, which storage medium is accessible by the processor 4. The mobile device 1 is further equipped with a communication unit 6 for communicating with external devices, such as other mobile devices or nodes of communication networks to which the mobile device 1 is connectable.

The mobile device 1 is further seen to comprise a display 7 for displaying information to a user, and, if realised in form of a touch-display, also for receiving information from the user in form of user input. The mobile device 1 may also comprise other known means for input and output of signals and information, such as buttons, microphones, loudspeakers etc.

The mobile device 1 is operable to perform all method steps of the inventive method, which method steps will be described in more detail below, through execution of a computer program stored in the storage medium 5.

The computer program stored on the mobile device 1 is realised in form of a mobile application, sometimes referred to as an app, which is a software application specifically designed to run on mobile devices such as smartphones and tablet computers. The mobile application is preferably downloadable into the storage medium 5 from a download server to which the mobile device 1 is connectable. The mobile application may further be adapted to a particular mobile operating system, such as Apple iOS, Google Android or Blackberry OS, and distributed through known application distribution platforms.

It should thus be appreciated that the terms "mobile application" and "app" hereinafter refers to a computer program in form of a mobile application which is stored on the storage medium 5 of the mobile device 1.

As illustrated in Fig. 1, the mobile device 1 stores a plurality of different mobile applications A1-A4, each represented by a box illustrating an icon that can be touched by a user to run the application. At least one of the mobile applications A1-A4 is intended to be used by people suffering from incontinence to help them monitor their daily use of hygiene products.

A hygiene product may herein be any product for retaining urine and/or faeces voided by a wearer of the product. In particular, the hygiene product may be an absorbent personal hygiene article, such as a male or female incontinence protector, a sanitary pad, a panty liner or a diaper, for example a diaper with tape fastener, a pant diaper or a belted diaper. The wearer of the hygiene product is the intended user of the mobile applications A1-A4 and may hereinafter be referred to as either "the product-wearer" or "the user".

The mobile applications A1-A4 are all configured to collect information that is relevant to the voiding behaviour of the user, which information herein is referred to as "voiding relevant information". Contrary to what is herein referred to as "voiding-related information", the voiding relevant information need not relate to the actual voiding behaviour of the user but may relate to anything that has impact on the voiding behaviour. The content of the voiding relevant information and the way it is collected may differ between the different applications A1-A4, as will be described in more detail below. The mobile applications A1-A4 may further be configured to provide product-related information as to the use of the hygiene product to the user, based on the voiding relevant information obtained by the respective mobile application. The product-related information typically includes recommendations on when to change the hygiene product.

With reference now made to Fig. 2a, the mobile applications A1-A4 are further configured to transmit the voiding relevant information to a cross-sharing database 8, in which it is stored. The cross-sharing database 8 may reside in a server 9 to which several mobile devices 1a, 1b running one or more of the mobile applications A1-A4 are communicatively connectable, such as an application server on the Internet. In this embodiment, the cross-sharing database 8 is a multi-user database storing voiding relevant information on multiple users of the mobile applications A1-A4.

In another embodiment, illustrated in Fig. 2b, the cross-sharing database 8 is a single-user database residing in the mobile device 1 itself. In this embodiment, the cross-sharing database 8 and the different mobile applications A1-A4 collecting voiding relevant information may all be stored on the storage medium 5 of the mobile device.

The voiding relevant information obtained by the mobile applications A1-A4 and stored in the cross-sharing database 8 may be any information that is relevant to the voiding behaviour of the user. It may comprise information on actual urinary and/or faecal insults by the user (i.e. voiding-related information), obtained by the different mobile applications A1-A4 through the use of sensors and/or through manual input of information. It may further comprise information on the user and/or user behaviours that affects or potentially affects the voiding behaviour of the user, also obtainable by means of various sensors of the mobile device 1 and/or through manual input of information into the different mobile applications.

As illustrated in Fig. 2a, the multi-user cross-sharing database 8 may comprise a plurality of tables T1-T6 in which the voiding relevant information is stored. In this exemplary embodiment, the following tables are included in the database:
- Users, T1: stores user data on users of the mobile applications A1-A4 and may for example include information on the age of the users, the gender of the users, etc.;
- Voiding, T2: stores voiding-related information on actual and/or predicted insults of urine and/or faeces by the users and may for example include information on the point in time of each insult, the type of the insult (i.e. urinary or faecal insult), the amount of each insult, etc.;
- Intake, T3: stores intake data on intakes of fluid and/or solid edible material by the users and may for example include information on the point in time of each intake, the type of the intake (i.e. fluid or solid), the amount of each intake, etc.;
- Health, T4: stores health data on the health of the users and may for example include information on diseases from which the users suffer, the use of medicines by the users, etc.;
- Exercise, T5: stores exercise data on the exercise routines of the users and may for example include information on when and for how long the users exercise, what type of activities are performed, etc.;
- Sleep, T6: stores sleep data on the sleep routines of the users and may for example include information on when and for how long the users sleep.

The single-user cross-sharing database 8 in Fig. 2b may comprise corresponding tables T1-T6 with the only difference that they only contain information on a single user.

As understood by the skilled person, the mobile applications A1-A4 obtaining the voiding relevant information may be any type of mobile applications and are not limited to mobile applications particularly intended to monitor the use of hygiene products. For example, the intake information may be obtained by an app for monitoring the diet of a user, the health information may be obtained by an app for monitoring the intake of medicines for one or more diseases from which the user suffers, the exercise information may be obtained by an exercise app for keeping track of the exercise routines of the user, and the sleep information may be obtained by an app for tracking the sleeping behaviour of the user, all configured to transmit the obtained information to the cross-sharing database 8 for subsequent use by a mobile application for monitoring the use of hygiene products.

Preferably, however, the mobile applications A1-A4 that obtain the voiding relevant information are also configured to monitor the use of hygiene products. In this case, the mobile applications A1-A4 form a set of mobile applications particularly intended to monitor the use of hygiene products, and to provide a user with recommendations as to said use. By sharing the information obtained by the different applications, the intelligence of the respective application can be increased and more well-founded recommendations as to the use of hygiene products can be provided to the user.

Preferably, both the mobile application that provides the product-related information to the product-wearer based on the voiding relevant information retrieved from the cross-sharing database, and the mobile application originally obtaining the voiding relevant information and providing it to the cross-sharing database, form part of a group comprising four mobile applications, hereinafter referred to as "Sleep Security", "Daily Log", "Pad Scanner" and "Reassure". These mobile applications are further developments of the mobile applications described in the co-pending patent applications PCT/SE2011/051565, PCT/SE2011/051566, PCT/SE2011/051551, and PCT/SE2011/051558, not available to the public at the date of filing of this application.

### Sleep Security

Sleep Security is a mobile application for monitoring use of a hygiene product based on movements of the user, i.e. the wearer of the hygiene product. In particular, as the name implies, Sleep Security is intended to monitor the use of the product while the user is sleeping. The application makes use of the findings that people suffering from incontinence are more inclined to void urine and faeces in light sleep phases. It also makes use of the fact that increased sleep movement is an indication of light sleep. By registering the sleep movement of the person wearing the absorbent product, product-related information that facilitates the use of the product can be derived and provided to the wearer of the product or to his caregiver.

To this end, Sleep Security registers the movement of the product-wearer by means of the movement sensing device 2 of the mobile device 1, evaluates whether the registered movement is indicative of urinary and/or faecal voiding by the product-wearer, and provides product-related information as to the use of the hygiene product based on the evaluation.

Fig. 3 illustrates an example of a signal *f*, hereinafter referred to as the awakeness signal, obtained by the Sleep Security application using the movement sensing device 2 of the mobile device 1. The awakeness signal *f* is used by Sleep Security to determine whether the product-wearer is likely to have voided urine and/or faeces, and, based on this determination, to provide the product-wearer or a caregiver of the product-wearer with recommendations as to the use of the absorbent product, for example a recommendation to change the product.

In an exemplary embodiment, Sleep Security is configured to assume that voiding takes place when the awakeness signal *f* rises above a certain threshold value, *Tr,* and to keep track of the number of assumed urinary and/or faecal insults by counting the number of times the awakeness signal rises above said threshold value. When the number of assumed urinary and/or faecal insults exceeds a maximum value, Sleep Security triggers an alarm to indicate to the product-wearer or his caregiver that it is time to change the hygiene product.

Furthermore, in accordance with the principles of the present invention, Sleep Security is configured to transmit any information that is relevant to the voiding behaviour of the product-wearer to the cross-sharing database 8. This information comprises at least information on the assumed urinary and/or faecal insults, e.g. the points in time at which the insults are assumed to have occurred, as determined from the registered movement of the user.

Sleep Security may further be configured to predict future voiding by the product-wearer, i.e. future insults of urine and/or faeces, based on one or more previously registered movements of the user, and to provide the product-related information to the product-wearer or his caregiver based on this prediction. To improve the predictions, Sleep Security may retrieve voiding relevant information obtained by other mobile applications from the cross-sharing database 8, and use this information together with the registered movement of the product-wearer in the prediction of future voiding.

For example, Sleep Security may be configured to retrieve intake and voiding information collected by the Daily Log application as further described below, and use this information in the prediction of future voiding. To this end, Sleep Security may be configured to analyse the relationship between intake and voiding by the user during daytime, as indicated by the information retrieved from the cross correlation database 8 and originally obtained by the Daily Log application, and the voiding behaviour of the user during night time, as indicated by the registered movement of the product-wearer. By analysing this relationship, preferably during a time period of several days and nights, the Sleep Security application can "learn" e.g. how many insults of urine that are likely to occur during a night following a day having a certain intake/voiding pattern. This knowledge can be used by Sleep Security in the prediction of future voiding of urine and/or faeces by the product-wearer.

Furthermore, the Sleep Security application can retrieve voiding-related information obtained by other mobile applications from the cross-sharing database 8, and compare this information with the registered movements of the product-wearer to adapt the algorithm used by Sleep Security for determining when urinary and/or faecal voiding is assumed to take place. For example, Sleep Security may retrieve information on the exact point in time at which urine or faeces is voided by the product-wearer, obtained by the Reassure application as described in more detail below, and compare this information with the registered awakeness curve *f*. In this way, the Sleep Security application may for example learn which awakeness curve patterns are indicative of urinary and/or faecal voiding, and which are not. Thereby, the algorithm used to determine when voiding has taken place can be adapted accordingly, e.g. by adapting the algorithm to exclude certain awakeness curve patterns, or by simply adjusting the threshold value *Tr.*

### Daily Log

Daily Log is a mobile application for monitoring use of an absorbent product based on intake and voiding information on a user, i.e. the wearer of the absorbent product.

The basic principles of the Daily Log application are illustrated in Fig. 4, showing the method steps performed by the mobile application upon execution thereof.

In a first step, S1, intake information indicative of intake of fluid and/or solid edible material by the product-wearer is obtained. In a second step, S2, voiding information indicative of urinary and/or faecal voiding by the product-wearer is obtained. The intake and voiding-related information is typically obtained through manual input of information into the mobile application, preferably by having the product-wearer enter the information into a daily log keeping track of the intake and voiding behaviours of the user. The intake information comprises at least an indication of the point in time for each intake, and the voiding information comprises at least an indication of a point in time for each insult of urine and/or faeces. Additionally, the intake information and the voiding information may comprise indications of the type and/or the amount of each intake and the type and/or the amount of each insult. In a third step, S3, future voiding by the product-wearer, i.e. future insults of urine and/or faeces, is predicted based on the intake and voiding information obtained in steps S1 and S2. In a fourth step, S4, product-related information as to the use of the absorbent product is provided to the product-wearer based on said prediction.

Furthermore, in accordance with the principles of the present invention, the Daily Log application is configured to transmit information that is relevant to the voiding behaviour of the product-wearer to the cross-sharing database 8. The transmitted information comprises at least information on the points in time of the insults registered by the product-wearer in the log, and preferably all of the above mentioned intake and voiding information.

The Daily Log application is also configured to retrieve voiding relevant information obtained by other mobile applications from the cross-sharing database 8, and to use this information together with the voiding and intake information in the daily log in order to improve the prediction of future insults.

For example, the Daily Log application may retrieve voiding relevant information indicative of the voiding behaviour of the product-wearer during night time, obtained by either or both of the Sleep Security application or the Reassure application (as described below). The Daily Log application may then compare the intake and voiding behaviours of the product-wearer during daytime with the voiding behaviour of the product wearer during night time. By conducting this comparison for a plurality of days and nights, the Daily Log application can learn how different intake/voiding patterns during day time affect the voiding behaviour of the product-wearer during night time. Thereby, the voiding relevant information obtained by the Sleep Security application can be used by the Daily Log application to improve the predictions of future insults.

### Pad Scanner

Pad Scanner is a mobile application for monitoring use of absorbent product based on analysis of images of used absorbent products. It is configured to determine a measure of absorption of fluid by the absorbent product based on the information in one or more images of the used absorbent product, and to display information relating to the use of the absorbent product on the mobile device on which the application is run.

Fig. 5 illustrates a used hygiene product 12 in form of an incontinence protector comprising an area 13 of absorbed fluid. The mobile device 1 captures an image of the product 12 by means of the image capturing device 3 of the mobile device 1, preferably during illumination of the product 12 by a light source 10 of the mobile device 1, e.g. an integrated camera flash. By analysing the colour an/or light intensity information of the pixels of the captured image, a degree of utilization of the absorbed product can be determined.

Furthermore, the Pad Scanner application may be configured to obtain information on the capacity of the absorbent product that is analysed, and to determine the amount (e.g. the volume) of the fluid absorbed by the absorbent product based on the analysis of the image and the known capacity of the product. In this way, information on the amount of urine and/or faeces voided by the product-wearer can be obtained. By keeping track of when the product-wearer changes product, e.g. through manual input of information indicating a change of product by the product-wearer, the Pad Scanner application can obtain information on when the product-wearer voids urine and/or faeces, and the amount of urine and/or faeces voided.

In accordance with the principles of the present invention, this information may be uploaded to the cross-sharing database 8 by the Pad Scanner application, in order to be used by other applications for monitoring use of hygiene products.

For example, the information may be used by the Daily Log application to obtain measures of the amount of urine and/or faeces voided between different points in time, which information can be compared with information on previous intakes of fluid and/or solid edible material to gain knowledge about the relationship between the intake behaviour and the voiding behaviour of the user. This knowledge can be used by the Daily Log application to adjust its prediction algorithms so as to more accurately predict future voiding by the product-wearer based on intake information input to the daily log by the product-wearer.

The information transmitted to the cross-sharing database 8 by the Pad Scanner application may also be used by the Sleep Security application. For example, the product-wearer may change to a new absorbent product before going to sleep and capture an image of the used absorbent product the next morning. The Pad Scanner application can then upload information on the amount voided by the product-wearer during the night, which information can be retrieved by the Sleep Security application and compared with the awakeness curve *f*, registered during the same night. In this way, the Sleep Security application can learn which awakeness curve patterns correspond to voiding by the product-wearer, and even establish a correlation between awakeness curve patterns and voided amounts. As described above, such knowledge can be used by the Sleep Security application to adapt the algorithm used to determine when voiding has taken place, e.g. by adapting it to exclude certain awakeness curve patterns or by adjusting the threshold value *Tr.* In fact, this knowledge may even be used by the Sleep Security application to estimate the actual volume of urine and/or faeces voided by the product-wearer through analysis of the awakeness curve alone.

### Reassure

Reassure is a mobile application for monitoring use of a hygiene product based on sensor signals obtained by an odour sensor device capable of detecting one or more chemicals indicative of a presence of urine and/or faeces in the hygiene product.

Fig. 6a illustrates a mobile monitoring system comprising a mobile device 1 running the Reassure application, and an odour sensor device 11. As illustrated in Fig. 6b, the odour sensor device 11 is preferably sized to be carried in a pocket of clothing of a wearer 17 of the hygiene product 12.

The odour sensor device 11 comprises an odour sensor 14, indicated in dashed lines, comprising one or more gas inlets 15 through which the gas to be analysed can enter the odour sensor. The odour sensor 14 may for example be configured to detect any or all of the chemicals, ammonia, amines, sulphides and ketones, all indicative of the presence of urine, and hydro dioxide, indole, skatole, thiols (sulfer) and hydrogen sulphide, all indicative of the presence of faeces.

Moreover, the odour sensor device 11 comprises a communication unit 16 for communicating with the Reassure application running on the mobile device 1, preferably over a wireless communication interface, such as a bluetooth interface. When the odour sensor 14 detects a chemical indicative of presence of urine and/or faeces, it generates a signal which is transmitted to the mobile device 1 by the communication unit 16. Preferably, the odour sensor 14 is configured to generate a signal in dependence of the concentration of the detected chemical, and hence send a signal that is indicative of the concentration of the detected chemical to the mobile device 1.

The signal received from the odour sensor device 11 is used by the Reassure application to determine the points in time when urinary and/or faecal voiding take place, and additionally also to determine the amount of urine and/or faeces voided, based on the concentration of the detected chemical.

In accordance with the principles of the present invention, this information may be uploaded to the cross-sharing database 8 by the Reassure application, in order to be used by other applications for monitoring use of hygiene products. For example, the information on when urinary and/or faecal voiding take place, and the information of the amount of urine and/or faeces voided by the product-wearer can be used by the Daily Log application and the Sleep Security application as described above.

As understood from the above description of various mobile applications, there are many different ways in which a mobile application can utilise voiding relevant information obtained by other mobile applications in order to provide recommendations as to the use of a hygiene product. It should thus be appreciated that the invention is not limited to the embodiments described above, but can be varied within the scope of the claims following hereinafter.

## Claims

1. A method performed through execution of a set of mobile applications for monitoring use of a hygiene product (12) worn by a wearer (17), which set of mobile applications is formed of a first mobile application and at least a second mobile application, the method comprises the steps of providing information related to the hygiene product based on the voiding behaviour of the product-wearer, and obtaining, by means of the at least a second mobile application, information that is relevant to the voiding behaviour of a wearer (17), and transmitting the obtained information to a database (8),
**wherein** the method further comprises the following steps performed through execution of the first mobile application in a mobile device (1) comprising a display for displaying information to a user:
- retrieving, from the database (8) shared by a plurality of different mobile applications (A1-A4), stored information that is relevant to the voiding behaviour of the wearer, obtained by means of at least the second mobile application being different than said first mobile application,
- obtaining, through said first mobile application, voiding-related information on the actual voiding behaviour of the product-wearer,
- comparing the voiding-related information with the information retrieved from the database (8),
- predicting a future voiding behaviour of the product-wearer (17) based on said comparison, and
- providing the product-related information related to a change of the hygiene product on the display of the mobile device based on said predicted future voiding behaviour,
wherein said first and second mobile applications are different mobile applications selected from a group of mobile applications (A1-A4) consisting of:
- a mobile application for obtaining information relevant to the voiding behaviour of a user or voiding-related information on the actual voiding behaviour of the product-wearer by analysing movements of the user, registered by means of a movement sensing device (2) of the mobile device (1);
- a mobile application for obtaining information relevant to the voiding behaviour of a user or voiding-related information on the actual voiding behaviour of the product-wearer through manual input on a mobile device (1) of at least information related to intakes of fluid and/or solid edible material by the user, and information related to voiding of urine and/or faeces by the user;
- a mobile application for obtaining information relevant to the voiding behaviour of a user or voiding-related information on the actual voiding behaviour of the product-wearer by analysing images of used absorbent products, captured by an image capturing device (3) of a mobile device (1), and
- a mobile application for obtaining information relevant to the voiding behaviour of a user or voiding-related information on the actual voiding behaviour of the product-wearer by analysing signals received from an odour sensor device (11) capable of detecting chemicals indicative of a presence of urine and/or faeces.

2. The method according to claim 1, wherein said voiding-related information is obtained by the first mobile application through manual input of information by the product-wearer (17) and/or by means of one or more sensor devices (2, 3, 11) for obtaining information on actual voiding by the product-wearer.

3. The method according to any of the previous claims, wherein the information retrieved from said database (8) comprises:
- data on the age and/or gender of the product-wearer;
- data on the health and/or use of medicines of the product-wearer;
- data on intakes of fluid and/or solid edible material by the product-wearer;
- data on exercise routines of the product-wearer, and/or
- data on sleep routines of the product-wearer.

4. The method according to any of claim 1 to 3, wherein the information retrieved from the database (8) comprises data on the actual voiding behaviour of the product-wearer (17), obtained by the at least second mobile application, the method further comprises the steps of:
- comparing said data with the voiding-related information obtained by the first mobile application, and
- using the result of the comparison in the prediction of the future voiding behaviour of the product-wearer.

5. The method according to any of the preceding claims, further comprising the steps of:
- obtaining capacity information related to the capacity of the hygiene product (12), and
- providing said product-related information based on said capacity information.

6. The method according to claim 5 when dependent on any of the claim 1 to 2, wherein the product-related information is based on a comparison between the predicted future voiding behaviour of the product-wearer (17) and said capacity information.

7. The method according to claim 6, further comprising the steps of:
- determining a maximum amount of urine and/or faeces that can be retained by the hygiene product (12), based on said capacity information, and
- determining the amount of urine and/or faeces likely to be voided by the product-wearer (17) before a future point in time, based on said predicted future voiding behaviour of the product-wearer, and
- providing the product-related information based on a comparison between said maximum amount and said amount likely to be voided before said future point in time.

8. The method according to claim 7, wherein both said maximum amount and said amount likely to be voided are determined as discrete numbers of urinary and/or faecal insults.

9. The method according to any of the preceding claims, wherein the product-related information is provided in form of visual, audible and/or vibratory signalling on the mobile device (1) of the product-wearer (17), and/or on a communication device to which the mobile device is communicatively connectable, e.g. a mobile device of a caregiver of the product-wearer.

10. The method according to any of the preceding claims, wherein said product-related information comprises any of, or any combination of: an indication that no change of product is necessary; an indication that urinary and/or faecal voiding has taken place and that it may be advisable to change the product; a recommendation to change the product; a recommendation to change the product before a certain time; and, a recommendation to exchange the product for another hygiene product having higher or lower capacity than the currently worn product.

11. The method according to any of the previous claims, wherein the hygiene product (12) is an absorbent personal hygiene article, such as a male or female incontinence protector, a sanitary pad, a diaper with tape fastener, a pant diaper or a belted diaper.

12. A set of computer programs in form of mobile applications (A1-A4) for monitoring use of a hygiene product (12) worn by a wearer (17), which set of computer programs is formed of a first mobile application and at least a second mobile application, **characterised in that** the set of computer programs are configured to share the information stored in a database (8), and the computer programs, when executed by a processor (4) of one or more mobile devices (1, 1a, 1b), causes the one or more mobile devices to perform the method according to any of the previous claims.

13. A computer-readable data carrier having stored thereon the set of computer programs of claim 12 and a database (8) configured to be shared by the first mobile application and the at least a second mobile application.

14. A mobile device (1) for monitoring use of a hygiene product (12) worn by a wearer (17), the mobile device comprising a processor (4), and a storage medium (5) for storing computer programs executable by said processor, **characterised in that** the storage medium (5) stores a set of computer programs according to claim 12.

15. A mobile device (1) according to claim 14, wherein said storage medium (5) stores the entire system of software components (A1-A4, 8) according to claim 13.

16. The mobile device (1) according to claim 14 or 15, further comprising:
- a movement sensing device (2) for registering the movements of the wearer when the mobile device (1) is carried by the wearer or placed next to the wearer in bed;
- an image capturing device (3) for capturing images of used absorbent products, and/or
- a communication unit (6) for communicating with an odour sensor device (11).

## Patentansprüche

1. Verfahren, das durch Ausführung eines Satzes mobiler Anwendungen zum Überwachen von Verwendung eines Hygieneprodukts (12) durchgeführt wird, das von einem Träger (17) getragen wird, welcher Satz von mobilen Anwendungen von einer ersten mobilen Anwendung und mindestens einer zweiten mobilen Anwendung gebildet ist, wobei das Verfahren die Schritte zum Bereitstellen von Informationen bezüglich des Hygieneprodukts, basierend auf dem Entleerungsverhalten des Produkt-Trägers, und Erhalten, mittels der mindestens einen zweiten mobilen Anwendung, von Informationen, die für das Entleerungsverhalten eines Trägers (17) relevant sind, und Übertragen der erhaltenen Informationen an eine Datenbank (8) umfasst,
**wobei** das Verfahren weiter die folgenden Schritte umfasst, die durch Ausführung der ersten mobilen Anwendung in einer mobilen Vorrichtung (1), die eine Anzeige zum Anzeigen von Informationen an einen Anwender umfasst, durchgeführt werden:
- Beziehen, von der Datenbank (8), die von einer Vielzahl unterschiedlicher mobiler Anwendungen (A1-A4) geteilt wird, gespeicherter Informationen, die für das Entleerungsverhalten des Trägers relevant sind, mittels mindestens der zweiten mobilen Anwendung erhalten, die sich von der ersten mobilen Anwendung unterscheidet,
- Erhalten, durch die erste mobile Anwendung, von entleerungsbezogenen Informationen über das tatsächliche Entleerungsveralten des Produkt-Trägers,
- Vergleichen der entleerungsbezogenen Informationen mit den Informationen, die von der Datenbank (8) bezogen werden,
- Vorhersagen eines zukünftigen Entleerungsverhaltens des Produkt-Trägers (17), basierend auf dem Vergleich, und
- Bereitstellen der produktbezogenen Informationen bezüglich einer Änderung des Hygieneprodukts auf der Anzeige der mobilen Vorrichtung, basierend auf dem vorhergesagten zukünftigen Entleerungsverhalten,
wobei die erste und zweite mobile Anwendung verschiedene mobile Anwendungen sind, ausgewählt aus einer Gruppe von mobilen Anwendungen (A1-A4), bestehend aus:
- einer mobilen Anwendung zum Erhalten von Informationen, die für das Entleerungsverhalten eines Anwenders relevant sind, oder entleerungsbezogenen Informationen über das tatsächliche Entleerungsverhalten des Produkt-Trägers, indem Bewegungen des Benutzers analysiert werden, die mittels einer Bewegungserfassungsvorrichtung (2) der mobilen Vorrichtung (1) registriert sind;
- einer mobilen Anwendung zum Erhalten von Informationen, die für das Entleerungsverhalten eines Benutzers relevant sind, oder entleerungsbezogenen Informationen über das tatsächliche Entleerungsverhalten des Produkt-Trägers durch manuelle Eingabe auf einer mobilen Vorrichtung (1) mindestens von Informationen, die sich auf Einnahmen von flüssigem und/oder festem essbaren Material durch den Benutzer beziehen, und Informationen, die sich auf Entleeren von Urin und/oder Fäkalien durch den Benutzer beziehen;
- einer mobilen Anwendung zum Erhalten von Informationen, die für das Entleerungsverhalten eines Benutzers relevant sind, oder entleerungsbezogenen Informationen über das tatsächliche Entleerungsverhalten des Produkt-Trägers, indem Bilder von verwendeten Absorptionsprodukten analysiert werden, die von einer Bildaufnahmevorrichtung (3) einer mobilen Vorrichtung (1) aufgenommen werden, und
- einer mobilen Anwendung zum Erhalten von Informationen, die für das Entleerungsverhalten eines Benutzers relevant sind, oder entleerungsbezogenen Informationen über das tatsächliche Entleerungsverhalten des Produkt-Trägers, indem Signale analysiert werden, die von einer Geruchssensorvorrichtung (11) empfangen werden, die imstande ist, Chemikalien zu detektieren, die eine Gegenwart von Urin und/oder Fäkalien angeben.

2. Verfahren nach Anspruch 1, wobei die entleerungsbezogenen Informationen von der ersten mobilen Anwendung durch manuelle Eingabe von Informationen durch den Produkt-Träger (17) und/oder mittels einer oder mehrerer Sensorvorrichtungen (2, 3, 11) zum Erhalten von Informationen über tatsächliche Entleerung durch den Produkt-Träger erhalten werden.

3. Verfahren nach einem der vorstehenden Ansprüche, wobei die Informationen, die von der Datenbank (8) bezogen werden, umfassen:
- Daten über das Alter und/oder Geschlecht des Produkt-Trägers;
- Daten über die Gesundheit und/oder Verwendung von Medikamenten des Produkt-Trägers;
- Daten über Einnahmen von flüssigem und/oder festem essbaren Material durch den Produkt-Träger;
- Daten über Trainingsgewohnheiten des Produkt-Trägers, und/oder
- Daten über Schlafgewohnheiten des Produkt-Trägers.

4. Verfahren nach einem der Ansprüche 1 bis 3, wobei die Informationen, die von der Datenbank (8) bezogen werden, Daten über das tatsächliche Entleerungsverhalten des Produkt-Trägers (17) umfassen, die von der mindestens zweiten mobilen Anwendung erhalten werden, wobei das Verfahren weiter die Schritte umfasst:
- Vergleichen der Daten mit den entleerungsbezogenen Informationen, die von der ersten mobilen Anwendung erhalten werden, und
- Verwenden des Ergebnisses des Vergleichs in der Vorhersage des zukünftigen Entleerungsverhaltens des Produkt-Trägers.

5. Verfahren nach einem der vorstehenden Ansprüche, weiter umfassend die Schritte:
- Erhalten von Kapazitätsinformationen bezüglich der Kapazität des Hygieneprodukts (12) und
- Bereitstellen der produktbezogenen Informationen, basierend auf den Kapazitätsinformationen.

6. Verfahren nach Anspruch 5, wenn von einem beliebigen der Ansprüche 1 bis 2 abhängig, wobei die produktbezogenen Informationen auf einem Vergleich zwischen dem vorhergesagten zukünftigen Entleerungsverhalten des Produkt-Trägers (17) und den Kapazitätsinformationen basieren.

7. Verfahren nach Anspruch 6, weiter umfassend die Schritte:
- Ermitteln einer Maximalmenge von Urin und/oder Fäkalien, die von dem Hygieneprodukt (12) zurückgehalten werden kann, basierend auf den Kapazitätsinformationen, und
- Ermitteln der Menge von Urin und/oder Fäkalien, die von dem Produkt-Träger (17) vor einem zukünftigen Zeitpunkt wahrscheinlich entleert wird, basierend auf dem vorhergesagten zukünftigen Entleerungsverhalten des Produkt-Trägers, und
- Bereitstellen der produktbezogenen Informationen, basierend auf einem Vergleich zwischen der Maximalmenge und der Menge, die wahrscheinlich vor dem zukünftigen Zeitpunkt entleert wird.

8. Verfahren nach Anspruch 7, wobei sowohl die Maximalmenge als auch die Menge, die wahrscheinlich entleert wird, als diskrete Zahlen von Urin- und/oder Fäkalinsulten ermittelt sind.

9. Verfahren nach einem der vorstehenden Ansprüche, wobei die produktbezogenen Informationen in Form von sichtbarer, hörbarer und/oder vibrierender Signalisierung auf der mobilen Vorrichtung (1) des Produkt-Trägers (17) und/oder auf einer Kommunikationsvorrichtung bereitgestellt sind, mit der die mobile Vorrichtung kommunikativ verbunden werden kann, z.B. eine mobile Vorrichtung eines Pflegers des Produkt-Trägers.

10. Verfahren nach einem der vorstehenden Ansprüche, wobei die produktbezogenen Informationen ein beliebiges oder eine beliebige Kombination umfassen von: einer Angabe, dass kein Wechsel vom Produkt nötig ist; einer Angabe, dass Urin- und/oder Fäkalienentleerung stattgefunden hat und dass es ratsam sein könnte, das Produkt zu wechseln; einer Empfehlung, das Produkt zu wechseln; einer Empfehlung, das Produkt vor einem gewissen Zeitpunkt zu wechseln; und einer Empfehlung, das Produkt für ein anderes Hygieneprodukt auszutauschen, das höhere oder niedrigere Kapazität aufweist als das aktuell getragene Produkt.

11. Verfahren nach einem der vorstehenden Ansprüche, wobei das Hygieneprodukt (12) ein absorbierender persönlicher Hygieneartikel ist, wie ein männlicher oder weiblicher Inkontinenzschutz, eine Damenbinde, eine Windel mit Klebebandverschluss, eine Hosenwindel oder eine Windel mit Gürtel.

12. Satz von Computerprogrammen in Form von mobilen Anwendungen (A1-A4) zum Überwachen von Verwendung eines Hygieneprodukts (12), das von einem Träger (17) getragen wird, welcher Satz von Computerprogrammen aus einer ersten mobilen Anwendung und mindestens einer zweiten mobilen Anwendung gebildet ist, **dadurch gekennzeichnet, dass** der Satz von Computerprogrammen konfiguriert ist, die Informationen, die in einer Datenbank (8) gespeichert sind, zu teilen und die Computerprogramme, wenn von einem Prozessor (4) einer oder mehrerer mobiler Vorrichtungen (1, 1a, 1b) ausgeführt, die eine oder mehreren mobilen Vorrichtungen veranlassen, das Verfahren nach einem der vorstehenden Ansprüche durchzuführen.

13. Computerlesbarer Datenträger, der den Satz von Computerprogrammen nach Anspruch 12 und eine Datenbank (8) darauf gespeichert aufweist, die konfiguriert sind, von der ersten mobilen Anwendung und der mindestens einen zweiten mobilen Anwendung geteilt zu werden.

14. Mobile Vorrichtung (1) zum Überwachen von Verwendung eines Hygieneprodukts (12), das von einem Träger (17) getragen wird, wobei die mobile Vorrichtung einen Prozessor (4) und ein Speichermedium (5) zum Speichern von Computerprogrammen, die von dem Prozessor ausgeführt werden können, umfasst, **dadurch gekennzeichnet, dass** das Speichermedium (5) einen Satz von Computerprogrammen nach Anspruch 12 speichert.

15. Mobile Vorrichtung (1) nach Anspruch 14, wobei das Speichermedium (5) das gesamte System von Softwarekomponenten (A1-A4, 8) nach Anspruch 13 speichert.

16. Mobile Vorrichtung (1) nach Anspruch 14 oder 15, weiter umfassend:
- eine Bewegungserfassungsvorrichtung (2) zum Registrieren der Bewegungen des Trägers, wenn die mobile Vorrichtung (1) von dem Träger getragen wird oder neben dem Träger im Bett platziert ist;
- eine Bildaufnahmevorrichtung (3) zum Aufnehmen von Bildern verwendeter Absorptionsprodukte, und/oder
- eine Kommunikationseinheit (6) zum Kommunizieren mit einer Geruchssensorvorrichtung (11).

## Revendications

1. Procédé réalisé par exécution d'un ensemble d'applications mobiles pour surveiller l'utilisation d'un produit d'hygiène (12) porté par un porteur (17), lequel ensemble d'applications mobiles est formé d'une première application mobile et d'au moins une deuxième application mobile, le procédé comprenant les étapes de fournir des informations relatives au produit d'hygiène sur la base du comportement d'évacuation du porteur de produit, et d'obtenir, au moyen de l'au moins une deuxième application mobile, des informations concernant le comportement d'évacuation d'un porteur (17), et de transmettre les informations obtenues à une base de données (8),
**dans lequel** le procédé comprend en outre les étapes suivantes réalisées par exécution de la première application mobile dans un appareil mobile (1) comprenant un écran pour afficher des informations à un utilisateur :
- récupérer, à partir de la base de données (8) partagée par une pluralité d'applications mobiles différentes (A1-A4), des informations stockées concernant le comportement d'évacuation du porteur, qui sont obtenues au moyen de l'au moins une deuxième application mobile qui est différente de ladite première application mobile,
- obtenir, via ladite première application mobile, des informations relatives à l'évacuation sur le comportement d'évacuation effectif du porteur de produit,
- comparer les informations relatives à l'évacuation avec les informations récupérées à partir de la base de données (8),
- prévoir un comportement d'évacuation futur du porteur de produit (17) sur la base de ladite comparaison, et
- fournir les informations relatives au produit associées à un changement du produit d'hygiène sur l'écran de l'appareil mobile sur la base dudit comportement d'évacuation futur prévu,
dans lequel lesdites première et deuxième applications mobiles sont des applications mobiles différentes choisies dans un groupe d'applications mobiles (A1-A4) constitué de :
- une application mobile permettant d'obtenir des informations concernant le comportement d'évacuation d'un utilisateur ou des informations relatives à l'évacuation sur le comportement d'évacuation effectif du porteur de produit en analysant les mouvements de l'utilisateur, qui sont enregistrés au moyen d'un dispositif de détection de mouvement (2) de l'appareil mobile (1) ;
- une application mobile permettant d'obtenir des informations concernant le comportement d'évacuation d'un utilisateur ou des informations relatives à l'évacuation sur le comportement d'évacuation effectif du porteur de produit par saisie manuelle sur un appareil mobile (1) d'au moins d'informations relatives à des ingestions de matières consommables liquides et/ou solides par l'utilisateur et d'informations relatives à une évacuation d'urine et/ou de matières fécales par l'utilisateur ;
- une application mobile permettant d'obtenir des informations concernant le comportement d'évacuation d'un utilisateur ou des informations relatives à l'évacuation sur le comportement d'évacuation effectif du porteur de produit en analysant des images de produits absorbants usagés, qui sont capturées par un dispositif de capture d'images (3) d'un appareil mobile (1), et
- une application mobile permettant d'obtenir des informations concernant le comportement d'évacuation d'un utilisateur ou des informations relatives à l'évacuation sur le comportement d'évacuation effectif du porteur de produit en analysant des signaux reçus depuis un dispositif de détection d'odeurs (11) capable de détecter des substances chimiques indicatives de la présence d'urine et/ou de matières fécales.

2. Procédé selon la revendication 1, dans lequel lesdites informations relatives à l'évacuation sont obtenues par la première application mobile par saisie manuelle d'informations par le porteur de produit (17) et/ou au moyen d'un ou plusieurs dispositifs de détection (2, 3, 11) permettant d'obtenir des informations sur l'évacuation effective par le porteur de produit.

3. Procédé selon l'une quelconque des revendications précédentes, dans lequel les informations récupérées à partir de ladite base de données (8) comprennent :
- des données sur l'âge et/ou le sexe du porteur de produit ;
- des données sur la santé et/ou l'utilisation de médicaments par le porteur de produit ;
- des données sur des ingestions de matières consommables liquides et/ou solides par le porteur de produit ;
- des données sur des programmes d'exercices du porteur de produit, et/ou
- des données sur des habitudes de sommeil du porteur de produit.

4. Procédé selon l'une quelconque des revendications 1 à 3, dans lequel les informations récupérées à partir de la base de données (8) comprennent des données sur le comportement d'évacuation effectif du porteur de produit (17), qui sont obtenues par l'au moins une deuxième application mobile, le procédé comprenant en outre les étapes de :
- comparer lesdites données avec les informations relatives à l'évacuation obtenues par la première application mobile, et
- utiliser le résultat de la comparaison dans la prévision du comportement d'évacuation futur du porteur de produit.

5. Procédé selon l'une quelconque des revendications précédentes, comprenant en outre les étapes de :
- obtenir des informations de capacité relatives à la capacité du produit d'hygiène (12), et
- fournir lesdites informations relatives au produit sur la base desdites informations de capacité.

6. Procédé selon la revendication 5 prise en dépendance avec l'une quelconque des revendications 1 à 2, dans lequel les informations relatives au produit sont basées sur une comparaison entre le comportement d'évacuation futur prévu du porteur de produit (17) et lesdites informations de capacité.

7. Procédé selon la revendication 6, comprenant en outre les étapes de :
- déterminer une quantité maximale d'urine et/ou de matières fécales pouvant être retenue par le produit d'hygiène (12), sur la base desdites informations de capacité, et
- déterminer la quantité d'urine et/ou de matières fécales susceptible d'être évacuée par le porteur de produit (17) avant un instant futur donné, sur la base dudit comportement d'évacutation futur prévu du porteur de produit, et
- fournir les informations relatives au produit sur la base d'une comparaison de ladite quantité maximale et ladite quantité susceptible d'être évacuée avant ledit instant futur donné.

8. Procédé selon la revendication 7, dans lequel ladite quantité maximale et ladite quantité susceptible d'être évacuée sont toutes deux déterminées comme des nombres discrets de souillures urinaires et/ou fécales.

9. Procédé selon l'une quelconque des revendications précédentes, dans lequel les informations relatives au produit sont fournies sous forme d'une signalisation visuelle, sonore et/ou vibratoire sur l'appareil mobile (1) du porteur de produit (17) et/ou sur un dispositif de communication auquel l'appareil mobile est connectable de manière communicative, par exemple un appareil mobile d'une personne qui s'occupe du porteur de produit.

10. Procédé selon l'une quelconque des revendications précédentes, dans lequel lesdites informations relatives au produit comprennent l'une quelconque de, ou toute combinaison de : une indication qu'aucun changement de produit n'est nécessaire ; une indication qu'une évacuation urinaire et/ou fécale a eu lieu et qu'il serait conseillé de changer de produit ; une recommandation de changer de produit ; une recommandation de changer de produit avant un certain temps ; et une recommandation de changer le produit pour un autre produit d'hygiène ayant une capacité supérieure ou inférieure à celle du produit actuellement porté.

11. Procédé selon l'une quelconque des revendications précédentes, dans lequel le produit d'hygiène (12) est un article d'hygiène personnelle absorbant, tel qu'une protection pour incontinence masculine ou féminine, une serviette hygiénique, une couche avec attache adhésive, une couche-culotte ou une couche à ceinture.

12. Ensemble de programmes informatiques sous forme d'applications mobiles (A1-A4) pour surveiller l'utilisation d'un produit d'hygiène (12) porté par un porteur (17), lequel ensemble de programmes informatiques est formé d'une première application mobile et d'au moins une deuxième application mobile, **caractérisé en ce que** l'ensemble de programmes informatiques est configuré pour partager les informations stockées dans une base de données (8), et les programmes informatiques, lorsqu'ils sont exécutés par un processeur (4) d'un ou plusieurs appareils mobiles (1, 1a, 1b), amènent l'un ou les plusieurs appareils mobiles à exécuter le procédé selon l'une quelconque des revendications précédentes.

13. Support de données lisible par ordinateur, comprenant stockés sur lui l'ensemble de programmes informatiques selon la revendication 12 et une base de données (8) configurée pour être partagée par la première application mobile et l'au moins une deuxième application mobile.

14. Appareil mobile (1) pour surveiller l'utilisation d'un produit d'hygiène (12) porté par un porteur (17), l'appareil mobile comprenant un processeur (4), et un support de stockage (5) pour stocker des programmes informatiques exécutables par ledit processeur, **caractérisé en ce que** le support de stockage (5) stocke un ensemble de programmes informatiques selon la revendication 12.

15. Appareil mobile (1) selon la revendication 14, dans lequel ledit support de stockage (5) stocke le système entier de composants logiciels (A1-A4, 8) selon la revendication 13.

16. Appareil mobile (1) selon la revendication 14 ou 15, comprenant en outre :
- un dispositif de détection de mouvement (2) pour enregistrer les mouvements du porteur lorsque l'appareil mobile (1) est porté par le porteur ou placé à côté du porteur alité ;
- un dispositif de capture d'images (3) pour capturer des images de produits absorbants usagés, et/ou
- une unité de communication (6) pour communiquer avec un dispositif de détection d'odeurs (11).
